# EUROPEAN PATENT APPLICATION

(11) **EP 3 566 741 A1**
(43) Date of publication of application: **13.11.2019**
(21) Application number: 18214606.8
(22) Date of filing: 20.12.2018
(51) Int. Cl.: A61M 25/10, A61M 1/10

(54) **INTRA-AORTIC DUAL BALLOON PUMP CATHETER DEVICE**

(30) Priority: 09.05.2018 CN 201810435347
(71) Applicant: Fuwai Hospital, Chinese Academy Of Medical Science, Beijing 100037 (CN)
(72) Inventor: YANG, Yuejin, Xicheng District, Beijing (CN); MAO, Yi, Xicheng District, Beijing (CN)
(74) Representative: Patentanwälte ter Smitten Eberlein-Van Hoof Rütten Partnerschaftsgesellschaft mbB

(57) **Abstract**

The present invention relates to an intra-aortic dual balloon pump catheter device. The intra-aortic dual balloon pump comprises: a catheter (2); a first balloon (4) and a second balloon (6) arranged to successively surrounding the catheter along the longitudinal direction of the catheter (2), wherein the position of the first balloon (4) is closer to a catheter end (5) of the catheter (2) than the position of the second balloon (6); the first balloon (4) and the second balloon (6) are periodically expanded to a dimension that blocks the aortic blood flow and contracted to a dimension that does not prevent the blood flow from passing through; wherein the first balloon (4) begins and finishes the inflation earlier than the second balloon (6) in each period of expansion and contraction.

## Description

The present invention relates to an intra-aortic dual balloon pump catheter device.

Intra-aortic balloon pump (IABP) is a mechanically assistive circulatory device widely and effectively used in the current clinical application. It is a cardiac catheterization therapy wherein a catheter with a balloon is implanted through the arterial system to an end proximal to the heart within the descending thoracic aorta, or an end distal to the left subclavian artery opening, inflating and deflating correspondingly to the cardiac cycle such that the blood changes in time phase within the aorta so as to mechanically assist the circulation. The aim is thus achieved to reduce aortic impendence and to increase aortic diastolic pressure while reducing myocardial oxygen consumption and increasing oxygen supply, so as to improve the function of the heart.

The intra-aortic balloon pump (IABP) may be applied to different situations to improve the patients hemodynamics by increasing coronary artery perfusion and reducing afterload of ventricle. These factors improve the heart function and myocardial oxygen demand-supply ratio. Its role is quite mild, and generally the cardiac output does not increase by more than 20%. The indications of intra-aortic balloon pump (IABP) include the following three situations: treatment of acute myocardial ischemia, postoperative cardiogenic shock, transition before cardiac transplantation, and etc.

The intra-aortic balloon pump device comprises a balloon catheter, and a counterpulsation machine serving as a driving section of the balloon catheter. The balloon catheter consists of an air supply catheter and a cylindrical balloon secured to the catheter. The standard for selecting the balloon catheter is to block 90%-95% of the aortic lumen after the balloon is inflated, and the balloon volume is greater than 50% of the heart's stroke volume. The counterpulsation machine comprises a monitoring part, a controlling part, a vacuum pump, and an air compressor. The air supply catheter communicates the cylindrical balloon with the vacuum pump and the air compressor. The monitoring part and the controlling part cooperates with each other to automatically identify the ECG or pressure signal according to the given parameters, automatically regulate the time phase of inflation and deflation, and automatically adjust the counterpulsation parameters so as to achieve the best counterpulsation effect, and automatically stop when there is a failure or abnormal stroke.

The balloon is inflated when the heart dilates, and the balloon is deflated when the heart contracts. The double effect of hemodynamics is thus produced. On one hand, the balloon is inflated in the heart dilation phase, so as to produce positive pressure for the blood to flow forwards, thereby improving the diastolic pressure and the coronary artery perfusion. In this case, the aortic valve closes in diastole while the balloon is inflated quickly to drive blood to both the distal and the proximal sides of the aorta, whereby the diastolic pressure at the aortic root is increased, and so are the coronary artery blood, myocardial oxygen supply, and whole body perfusion. The increase of the diastolic pressure greatly lowers the work load of the heart, particularly the left ventricle, and the blood supply is greatly improved. On the other hand, the balloon is deflated before the systole to lower the systolic pressure (cardiac afterload), so as to produce negative pressure, whereby the aortic pressure is reduced instantly, the ejection resistance and the cardiac afterload lowered, the cardiac output increased, and the myocardial oxygen consumption reduced. The decrease in ejection resistance improves the left ventricular ejection. In this case, the blood is inhaled into the blood chamber, the aortic pressure is lowered and the cardiac afterload is reduced. The controlling part is able to adjust the size of the balloon by controlling the air amount entering the balloon.

The counterpulsation of the balloon is usually triggered by the arterial waveform recorded at the top of the balloon, or is controlled by the electrocardiogram QRS waveform with time. The balloon counterpulsation must be inflated and deflated within accurate time period. Ideally, the balloon shall be inflated when the aortic valve is just closed, corresponding to the trace between two ascending waves of arterial waveform. Meanwhile, it is preferable that the balloon is deflated before the left ventricle is about to eject. Premature deflation in diastole may bring poor efficacy.

The current clinical IABP catheter can be only inflated and deflated by its balloon corresponding to the cardiac cycle to cause change in time phase of the blood within the aorta, but cannot actively drive the blood.

Therefore, it is required to provide an IABP catheter which may further improve the blood supply.

Said technical problem may be solved by the present invention providing an intra-aortic dual balloon pump catheter device. The intra-aortic dual balloon pump catheter device comprises: a catheter; a first balloon and a second balloon, the first balloon and the second balloon being arranged to successively surrounding the catheter along the longitudinal direction of the catheter, wherein the position of the first balloon is closer to a catheter end than the position of the second balloon; a monitoring part, for monitoring the cardiac cycle and the arterial pressure of the catheter end; air pumps, respectively mated with the first balloon and the second balloon for supplying and withdrawing air; a first intake pipe and a second intake pipe, one end of which is in communication with the first balloon and the second balloon respectively, and the other end of which is in communication with the respectively mated air pump; a controlling part, adapted to control the air pumps to inflate and deflate the first balloon and the second balloon according to the cardiac cycle and the arterial pressure of the catheter end monitored by the monitoring part, such that the first balloon and the second balloon are periodically expanded to a dimension that blocks the aortic blood flow and contracted to a dimension that does not prevent the blood flow from passing through; wherein the air pumps are controlled such that the first balloon begins and finishes the inflation earlier than the second balloon in each period of expansion and contraction.

In diastole, the first balloon begins and finishes the inflation earlier than the second balloon, which brings the following effects: on one hand, the inflation of the first balloon per se drives the blood to both the distal and the proximal sides of the aorta, so that the diastolic pressure at the aortic root is increased, thereby increasing the coronary artery blood and myocardial oxygen supply, while promoting the blood perfusion to the whole body; on the other hand, due to the time difference in the inflation process between the first balloon and the second balloon, the first balloon that is firstly inflated blocks the upstream blood and squeezes the downstream blood, and the second balloon that is subsequently inflated further enhances the squeezing effect on the downstream blood because the upstream has already been blocked by the first balloon, and thus the inflation of the second balloon produces a significant pushing effect towards ahead.

According to an embodiment of the intra-aortic dual balloon pump catheter device according to the present invention, the air pumps are controlled such that the second balloon begins to be inflated after the first balloon finishes inflation. However, in an alternative embodiment, the air pumps may be controlled such that the second balloon begins to be inflated before the first balloon finishes inflation. In either of the above embodiments, since the first balloon firstly finishes the inflation so as to block the upstream blood, the second balloon provides better squeezing effect on the downstream blood.

According to another embodiment of the intra-aortic dual balloon pump catheter device according to the present invention, the air pumps are controlled such that the first balloon and the second balloon are contracted at the same time.

In systole, preferably before the systole is about to begin, the first balloon and the second balloon are contracted at the same time, the blood is withdrawn by the negative pressure and enters into the cavity produced by the contraction, whereby the ejection resistance and the cardiac afterload are lowered, and the cardiac output is increased.

The first balloon and the second balloon are in communication with the respectively mated air pump via a first intake pipe and a second intake pipe. There may be multiple embodiments for the arrangement of the intake pipes and the catheter.

According to an embodiment of the intra-aortic dual balloon pump catheter device according to the present invention, the first intake pipe surrounds the catheter and extends together therewith through the second balloon. Preferably, after extending through the second balloon, the first intake pipe and the catheter surrounded by the first intake pipe extend in parallel with the second intake pipe. Or, in another preferable embodiment, after extending through the second balloon, the first intake pipe is surrounded by and extends together with the second intake pipe.

According to another embodiment of the intra-aortic dual balloon pump catheter device according to the present invention, after extending through the second balloon respectively, the catheter and the first intake pipe extend in parallel with the second intake pipe.

According to an embodiment of the intra-aortic dual balloon pump catheter device according to the present invention, the first balloon has a length less than that of the second balloon. The first balloon and the second balloon play different roles in the process of successive actions. The first balloon is mainly to block the upstream blood, in addition to driving the blood to flow upstream and downstream by its own volume expansion, so as to create conditions for the inflation of the second balloon to squeeze the blood to move downstream. The volume expanded by inflation of the second balloon determines the effect of squeezing the blood to move downstream. A longer second balloon helps to squeeze more blood downstream and has a more apparent promoting effect.

Preferably, the first balloon has a length that is one-tenth of a total length of the first balloon and the second balloon.
Fig. 1 shows an intra-aortic balloon pump catheter device of the prior art, which has only one balloon;
Fig. 2 shows a partial section view of an intra-aortic dual balloon pump catheter device according to the first embodiment of the present invention;
Fig. 3 shows a partial section view of an intra-aortic dual balloon pump catheter device according to the second embodiment of the present invention;
Fig. 4 shows a cross section view seen from line A-A in Fig. 3.

### List of reference sign

1 guide wire
2 catheter
3 first intake pipe
4 first balloon
5 catheter end
6 second balloon
7 second intake pipe

The intra-aortic dual balloon pump catheter device of the present invention will be described in detailed embodiments with reference to the accompanying drawings. It shall be noted that the accompanying drawings are given by way of illustration only, and shall not be construed as limiting the present invention.

The intra-aortic dual balloon pump catheter device is guided by a guide wire 1 of a catheter 2 and reaches a predetermined position within the aorta, and then the guide wire 1 exits from the catheter 2.

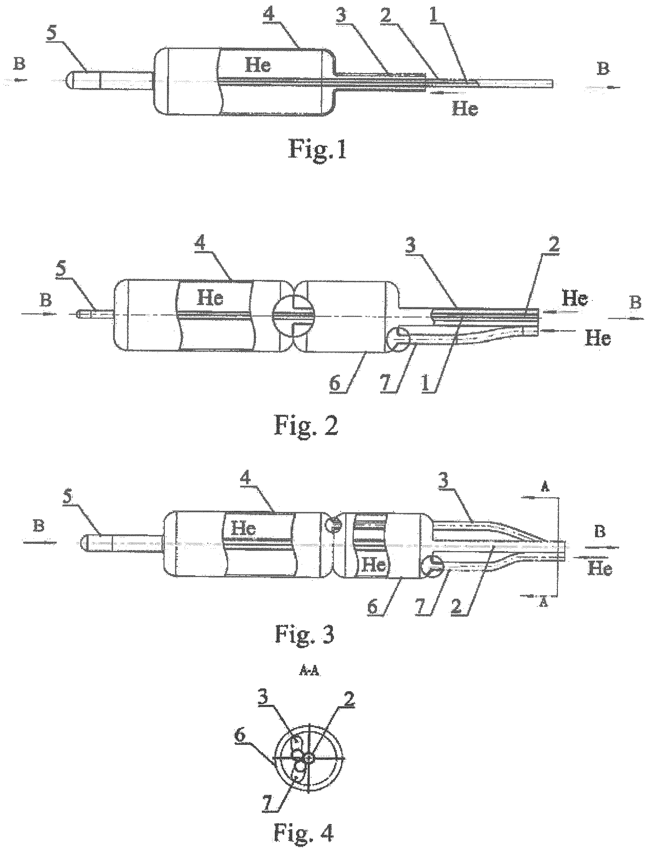

Fig. 2 shows a partial section view of an intra-aortic dual balloon pump catheter device according to the first embodiment of the present invention. As shown in the figure, a first balloon 4 and a second balloon 6 are arranged to successively surrounding the catheter 2 along the longitudinal direction of the catheter 2, wherein the position of the first balloon 4 is closer to a catheter end 5 than the position of the second balloon 6. A first intake pipe 3 and a second intake pipe 7 have one end in communication with the first balloon 4 and the second balloon 6 respectively, and the other end in communication with the air pump (not shown) respectively mated with the balloons and used for supply and withdrawal of air.

The intra-aortic dual balloon pump catheter device according to the first embodiment of the present invention further comprises a monitoring part and a controlling part that are not shown in the figure. The monitoring part is used for monitoring the cardiac cycle and the arterial pressure of the catheter end 5, while the controlling part is adapted to control the air pumps to inflate and deflate the first balloon 4 and the second balloon 6 according to the cardiac cycle and the arterial pressure of the catheter end 5 monitored by the monitoring part, such that the first balloon 4 and the second balloon 6 are periodically expanded to a dimension that blocks the aortic blood flow and contracted to a dimension that does not prevent the blood flow from passing through.

In this embodiment, the first intake pipe 3 protruding from the first balloon 4 surrounds and extends together with the catheter 2 through the second balloon 6, and then extends in parallel with the second intake pipe 7 protruding from the second balloon 6. An air pump mated with the first intake pipe 3 and the second intake pipe 7 respectively may supply, for example, helium gas into the first balloon 4 and the second balloon 6 to inflate the latter. The whole process of inflation is required to be completed within 130 ms. However, the air pumps are controlled such that the first balloon 4 begins and finishes the inflation earlier than the second balloon 6 in each period of expansion and contraction.

In diastole, the first balloon 4 begins and finishes the inflation earlier than the second balloon 6. The inflation of the first balloon 4 per se drives the blood to both the distal and the proximal sides of the aorta, so that the diastolic pressure at the aortic root is increased, thereby increasing the coronary artery blood and myocardial oxygen supply, while promoting the blood perfusion to the whole body. Then, the first balloon 4 that is firstly inflated blocks the upstream blood and squeezes the downstream blood, and the second balloon 6 that is subsequently inflated further enhances the squeezing effect on the downstream blood because the upstream has already been blocked by the first balloon 4.

Before the systole is about to begin, the air pump withdraws the air from the first balloon 4 and the second balloon 6 so that the balloons are contracted at the same time. The blood is withdrawn by the negative pressure and enters into the cavity produced by the contraction, whereby the ejection resistance and the cardiac afterload are lowered, and the cardiac output is increased.

Herein, the air pumps are controlled such that the second balloon 6 begins to be inflated after the first balloon 4 finishes inflation. However, the air pumps may also be controlled such that the second balloon 6 begins to be inflated before the first balloon 4 finishes inflation. The desired technical effect may be achieved as long as the first balloon 4 finishes the inflation process prior to the second balloon 6.

Fig. 3 shows a partial section view of an intra-aortic dual balloon pump catheter device according to the second embodiment of the present invention. Fig. 4 shows a cross section view seen from line A-A in Fig. 3. It differs from the first embodiment of the present invention in that: the first intake pipe 3 protruding from the first balloon 4 does not surround the catheter 2 through the first balloon 4 and the second balloon 6, but passes through the second balloon 6 individually. The first intake pipe 3 through the second balloon 6, the catheter 2, and the second intake pipe 7 protruding from the second balloon 6 extend in parallel with each other as shown in the figure.

While the present invention has been described with reference to the preferred embodiments, the spirit and scope of the invention are not limited to the disclosure herein. According to the teaching of the present invention, those skilled in the art are able to deduce more embodiments and applications without departing from the spirit and scope of the present invention, which are not defined by the embodiments but by the appended claims.

## Claims

1. An intra-aortic dual balloon pump catheter device, which comprises:
a catheter (2);
a first balloon (4) and a second balloon (6), arranged to successively surrounding the catheter (2) along the longitudinal direction of the catheter (2), wherein the position of the first balloon (4) is closer to a catheter end (5) of the catheter (2) than the position of the second balloon (6);
a monitoring part, for monitoring the cardiac cycle and the arterial pressure of the catheter end (5);
air pumps, respectively mated with the first balloon (4) and the second balloon (6), for supplying and withdrawing air;
a first intake pipe (3) and a second intake pipe (7), one end of which is in communication with the first balloon (4) and the second balloon (6) respectively, and the other end of which is in communication with the respectively mated air pump;
a controlling part, adapted to control the air pumps to inflate and deflate the first balloon (4) and the second balloon (6) according to the cardiac cycle and the arterial pressure of the catheter end (5) monitored by the monitoring part, such that the first balloon (4) and the second balloon (6) are periodically expanded to a dimension that blocks the aortic blood flow and contracted to a dimension that does not prevent the blood flow from passing through;
wherein the air pumps are controlled such that the first balloon (4) begins and finishes the inflation earlier than the second balloon (6) in each period of expansion and contraction.

2. The intra-aortic dual balloon pump catheter device according to Claim 1, **characterized in that** the air pumps are controlled such that the second balloon (6) begins to be inflated after the first balloon (4) finishes inflation.

3. The intra-aortic dual balloon pump catheter device according to Claim 1, **characterized in that** the air pumps are controlled such that the second balloon (6) begins to be inflated before the first balloon (4) finishes inflation.

4. The intra-aortic dual balloon pump catheter device according to Claim 1, **characterized in that** the air pumps are controlled such that the first balloon (4) and the second balloon (6) are contracted at the same time.

5. The intra-aortic dual balloon pump catheter device according to Claim 1, **characterized in that** the first intake pipe (3) surrounds the catheter (2) and extends together therewith through the second balloon (6).

6. The intra-aortic dual balloon pump catheter device according to Claim 5, **characterized in that**, after extending through the second balloon (6), the first intake pipe (3) and the catheter (2) surrounded by the first intake pipe (3) extend in parallel with the second intake pipe (7).

7. The intra-aortic dual balloon pump catheter device according to Claim 1 or 5, **characterized in that**, after extending through the second balloon (6), the first intake pipe (3) is surrounded by and extends together with the second intake pipe (7).

8. The intra-aortic dual balloon pump catheter device according to Claim 1, **characterized in that**, after extending through the second balloon (6) respectively, the catheter (2) and the first intake pipe (3) extend in parallel with the second intake pipe (7).

9. The intra-aortic dual balloon pump catheter device according to Claim 1, **characterized in that** the first balloon (4) has a length less than that of the second balloon (6).

10. The intra-aortic dual balloon pump catheter device according to Claim 1, **characterized in that** the first balloon (4) has a length that is one-tenth of a total length of the first balloon (4) and the second balloon (6).
